Europäisches Patentamt

(19) European Patent Office

Office européen des brevets

(11) Publication number : **0 404 376 B1**

(12) **EUROPEAN PATENT SPECIFICATION**

(45) Date of publication of patent specification :
23.03.94 Bulletin 94/12

(51) Int. Cl.⁵ : **A61K 31/415, A61K 47/12**

(21) Application number : **90306026.7**

(22) Date of filing : **05.06.90**

(54) **Buffered metronidazole compositions for intravaginal treatment of vaginal infections.**

(30) Priority : **06.06.89 US 362273**

(43) Date of publication of application :
**27.12.90 Bulletin 90/52**

(45) Publication of the grant of the patent :
**23.03.94 Bulletin 94/12**

(84) Designated Contracting States :
**AT BE CH DE DK ES FR GB GR IT LI LU NL SE**

(56) References cited :
**EP-A- 0 024 023**
**EP-A- 0 241 175**
**EP-A- 0 257 007**

(73) Proprietor : **CURATEK PHARMACEUTICALS**
**Limited Partnership**
**1965 Pratt Boulevard**
**Elk Grove Village, IL 60007 (US)**

(72) Inventor : **Borgman, Robert J.**
**1735 Victoria Road**
**Mundelein, Illinois 60060 (US)**

(74) Representative : **Heath, Derek James et al**
**BROMHEAD & CO. 19 Buckingham Street**
**London WC2N 6EF (GB)**

Jouve, 18, rue Saint-Denis, 75001 PARIS

## Description

This invention relates to a composition for intravaginal treatment of bacterial vaginosis and trichomoniasis with metronidazole formulations buffered to physiological vaginal pH.

Bacterial vaginosis (BV) is associated with an increased volume of vaginal discharge which has a foul, fishy odor. Vaginal pH is elevated from the normal range (pH 3-4) to values $\geq$ pH 4.7. The odor and elevated pH are caused by a high level of amines, most notably trimethylamine, in the vagina. These amines are volatilized when the pH is raised, for example, as with addition of KOH or interaction with semen. The vaginal discharge is homogenous in appearance as opposed to the flocculent discharge seen in Candida vaginitis. In contrast to candidiasis and trichomoniasis, itching generally is not associated with BV. A microscopic examination of a wet mount of the vaginal discharge in BV reveals an absence of polymorphonuclear leukocytes (PMNs). In contrast, the presence of many PMNs in a vaginal discharge is indicative of trichomoniasis, gonorrhea, or chlamydial cervicitis.

The causative organism for BV is a matter of some controversy. Gardnerella vaginalis is isolated from 98% of women with BV, but is also recovered in smaller numbers as normal flora in the vagina of asymptomatic women in incidences as high as 68% (Totten et al, 1982).

In those conditions where Gardnerella is present in higher concentrations, there is a significant decrease in the numbers of Lactobacilli present compared to the normal vagina. The normal vaginal flora is composed predominantly of Lactobacillus species, with an average pH of 4.0 (Hill and Embil, 1986; Bartlett and Polk, 1984). This low pH fosters growth and maintenance of the acidophilic Lactobacilli (anaerobic and facultatively anaerobic Gram-positive bacilli) that dominate the normal flora in concentrations of $10^8$ to $10^9$ Lactobacilli per milliliter of vagina secretions (Larsen and Galask, 1982; Rein, 1985). It is not known if a decrease in the Lactobacilli allows the Gardnerella to multiply, or if the increased numbers of Gardnerella actually inhibit the Lactobacilli. In any event, if the predominant microorganism present in the wet mount is not Lactobacilli, then BV must be suspected.

There have been overgrowths of other microorganisms seen in BV. Mycoplasma hominis and anaerobic bacteria including Bacteroides, Peptococcus, and Mobiluncus are also highly associated with BV (Eschenbach et al, 1988). In BV, G. vaginalis and the anaerobes can be present in overgrowths 1000 to 100,000 times more frequently than normal. It is also not known if the anaerobes are a result of the decreased amounts of Lactobacilli, or if they are responsible for the decrease. These organisms are present, however, in concentrations that should be considered pathogenic (Mead et al, 1986).

Characteristically seen in the wet mount in BV are abnormal cells termed "clue cells." These clue cells are vaginal epithelial cells with such a heavy coating of bacteria surrounding them that their peripheral borders are obscured (Eschenbach et al. 1988).

Peeters and Piot (1985) developed an experimental model of the G. vaginalis adherence to vaginal epithelial cells forming "clue cells." Using this model they found that the optimum pH for adhesion in vitro was pH 5 to 6 (the vaginal pH of women with bacterial vaginosis) and adhesion was limited at pH 3 to 4 which is the normal pH of vaginal fluid in women without vaginosis. If the same is true in vivo, a rise in vaginal pH is possibly a prerequisite in the pathogenesis of BV and perhaps precedes the formation of the pathognomonic "clue cells."

The antibacterial activity of Lactobacilli against other microorganisms has been suggested (Mardh and Soltesy, 1983). Skavin and Sylwan (1986) found that Lactobacilli strains inhibited growth of bacterial strains implicated in and isolated from women with BV in in vitro cultures. The bacterial strains tested included Mobiluncus mulieris, Mobiluncus curtisii, G. vaginalis, Peptococcus species, Peptococcus asaccharolyticus, Peptostreptococcus anaerobius, Gram-positive anaerobic coccus, and Bacteroides species. They also found that the lowest pH which would allow macroscopically visible growth of these bacterial strains ranged from pH 5.0 to 5.5. This data supports the importance of establishing and maintaining the presence of the Lactobacillus-dominated normal vaginal flora and the necessary pH environment for their growth and inhibition of other BV associated bacteria.

A clinical diagnosis of BV is made if three or more of the following four clinical criteria are present: (1) a homogenous discharge; (2) a pH $\geq$ 4.7; (3) a "fishy" amine odor upon the addition of 10% KOH to discharge; (4) presence of epithelial clue cells representing greater than or equal to 20% of vaginal epithelial cells (Eschenbach et al, 1988).

The efficacy of metronidazole in the treatment of BV as well as trichomoniasis is known. A marked effectiveness (essentially 100%) for metronidazole, given at 500 mg by mouth, twice daily for seven days has been demonstrated. Cure rates of 80-90% have repeatedly been reported since that time by the oral route of administration (Pheiffer et al., 1978; Balsdon et al., 1980; Eschenbach et al., 1983; Purdon et al., 1984; Charles et al., 1985; Swedberg et al., 1985; Malouf et al., 1981; Amsel et al., 1982; Hagstrom and Lindstedt, 1983; Mead et al., 1986). These studies employed the oral use of metronidazole in doses that ranged from 400 to 500 mg twice daily for three to seven days or 2 grams in a single dose. Heretofore, it has been generally accepted that the oral administration of metronidazole for five to seven days is the most effective way to treat BV; however, such a treatment for BV is not approved by the United States Food and Drug Administration (FDA). The Center for Disease Control recommends a dose of 500 mg of metronidazole given twice daily for seven days for treatment of bacterial vaginosis (CDC, 1985). Only one published paper reports the use of intravaginal metronidazole therapy for BV (Bistoletti et al., 1986). The authors compared the oral treatment which consisted of 400 mg of metronidazole twice daily for seven days to the application of a vaginal tablet containing 500 mg of metronidazole once daily for seven days.

The Merck Manual (15th edition, 1987) states on p. 244 that orally administered metronidazole provides effective female therapy when given at a single dose level of two grams, although the drug can be administered by injection.

The adverse reactions from oral administration of metronidazole can be extensive, however. For metronidazole, the "Modern Drug Encyclopedia" [A.J. Lewis, Editor, pub. by Vocke Medical Books, New York, N.Y. (1979)], contains the following statement on metronidazole:

"Adverse Reactions: Nausea, headache, anorexia, vomiting, diarrhea, epigastric distress, abdominal cramping, constipation, a metallic, sharp and unpleasant taste, furry tongue, glossitis, stomatitis, leukopenia, dizziness, vertigo, incoordination, ataxia, convulsive seizures, numbness or paresthesia of extremities, fleeting joint pains, confusion, irritability, depression, insomnia, mild erythematous eruption, weakness, urticaria, flushing, dryness of the mouth, vagina or vulva, pruritus, dysuria, cystitis, sense of pelvic pressure, dyspareunia, fever, polyuria, incontinence, decrease of libido, nasal congestion, proctitis, pyuria, and rarely, an unexplained darkening in the color of the urine have been reported. Flattening of the T wave may be seen in electrocardiographic tracings."

The need for providing safe and effective treatment for BV (without, for example, the side effects associated with the oral usage of metronidazole) assumes a more acute and pressing status when epidemiological trends and possible sequelae of a serious nature are given consideration. For example, vaginal infection with G. vaginalis, has been associated with possible sequelae, such as pelvic inflammatory disease, endometritis, and premature labor (Mead et al., 1986) that have an attendant, significant morbidity profile. Although there is no direct evidence linking BV with these conditions, it is not unreasonable to assume that an overgrowth of 10,000 to 100,000 anaerobic organisms in the vagina may result in certain genital diseases (Mead et al, 1986). Moreover, in the last decade there has been a tendency towards a reduction in gonorrhea and trichomoniasis while, during the same time span, there has been an increase in the so called "non-specific genital disease" (Staerfelt et al, 1983). Further, BV may account for significantly more total vaginitis patients than either Candida or trichomoniasis (Mead et al, 1986).

Since BV is a localized problem, intravaginal application of metronidazole should in principle be clinically effective. Moreover, since in intravaginal application, unaffected organ systems would be subjected to significantly lower or non-detectable levels of metronidazole, its side effects would be therefore minimized or eliminated.

A desirable treatment for BV would be an intravaginal composition that delivers a minimum effective dose of metronidazole while it simultaneously adjusts and maintains the vaginal pH at about the normal physiological range.

An ideal treatment for BV would therefore be a formulation which would deliver an antimicrobial agent directly to the vagina while simultaneously adjusting and maintaining the vaginal pH to the normal physiological range.

Intravaginal metronidazole therapy for BV has been studied (Bistoletti et al., 1986). The authors compared oral treatment which consisted of 400 mg of metronidazole in the morning and evening for seven days to vaginal treatment consisting of the application of a vaginal insert containing 500 mg of the drug every evening for seven days. Thus, the total dose given was 5.6 g in the oral, and 3.5 g in the vaginal, treatment groups. The findings in the 38 patients who completed the study showed a cure rate, at four weeks after initiation of therapy, to be 15 out of 19 (79%) for the vaginal treatment group and 14 out of 19 (74%) after oral treatment. Cure was based on assessment of pH, vaginal discharge, the 10% KOH amine test, and examination of a wet smear for clue cells. These same authors also reported that lactate-producing microorganisms (Lactobacilli and aerobic Streptococci) were found more frequently after vaginal than after oral treatment and speculated that this difference may be due to the higher local concentration of the drug achieved by intravaginal administration. In this regards a low concentration of metronidazole has been found in the vaginal fluid after a single oral dose of 2 g metronidazole (Davis et al., 1984). These authors concluded that topical administration of metronidazole might be more effective in re-establishing the normal microflora in the vagina. No side effects were reported related to the intravaginal use of metronidazole as the 500 mg insert. Although this study showed effectiveness of vaginally administered metronidazole, these researchers still used a high dose (3.5 grams) and made no attempt to adjust and control vaginal pH.

Like BV, Trichomonas vaginalis infections in symptomatic women cause complaints of abnormal discharge and odor in addition to pruritus, dyspareunia, or dysuria (Hager et al, 1980). Diagnosis requires identification of organisms by microscopic examination of the discharge, presence of a gray or yellow-green discharge, pH of discharge above 4.5, and a positive sniff test for odor producing volatile polyamines (McCue, 1989). An elevated vaginal pH encourages the growth of trichomonads. Foute and Kraus (1980) reported a vaginal pH above 4.5 to be associated and indicative of a trichomonal infection. Treatment generally consists of oral metronidazole therapy that is FDA approved. Topical therapy is considered less effective, however. (Robbie &Sweet, 1983; mcCue, 1989).

Where failure of treatment of a resistant case by oral metronidazole is encountered, a combination of oral and topical (vaginally applied) metronidazole has been recommended (Fouts and Kraus, 1980). These authors recommend a total dose from 14 grams to as high as 42 grams of oral metronidazole combined with a 500 mg vaginal dose daily or every other day for up to 14 days. Clearly, an alternate to this extremely high dosing is desirable.

Because of low water solubility of metronidazole, various oil-based metronidazole compositions have been developed, which are generally either creams (oil in water emulsions) or ointments (petroleum jelly based compositions) with metronidazole being dissolved/suspended in the oil/water phases.

Romanian Patent No. 80,363, published November 30, 1982 (reported also at C.A. 101:116743c), describes a vaginal gel with antibiotic and anti-inflammatory activity. This gel comprises metronidazole, nystatin with other antibacterials selected from nitrofural, chloramphenicol, and tetracycline and camazulene or hexoestrol acetate incorporated into Carbopol 940™, a gel-forming polyacrylic acid polymer available from B.F. Goodrich, Cincinnati, Ohio.

Such gel formulation suffers from the disadvantage that it includes, in addition to metronidazole, various active antibiotic, antimicrobial and antimycotic agents. Such gel formulation then operates intravaginally on a broad spectrum "shot gun" basis to destroy not only the harmful bacteria associated with "vaginitis," but also the desirable bacteria, such as the Lactobacilli and other lactate-producing organisms (e.g., aerobic Streptococci) that are present in the normal vagina. In addition, the Romanian patent teaches a gel formulation for intravaginal use which is formulated at a pH of 6 to 6.5. Hence, use of such a vaginal gel formulation is open to question from the standpoint of being a safe treatment for BV or trichomoniasis since it leaves the treated vagina in an abnormal condition where reinfection or infection by other opportunistic microorganisms are possible sequelae.

A known commercial vaginal formulation of metronidazole currently on the international market for use as a trichomonacide, but not in the United States, is produced by Rhone-Poulenc Pharma Inc. of Montreal, P.Q., Canada. This formulation is a cream which contains 500 mg of metronidazole per application (5 grams). The recommended dose for trichomoniasis is one application once or twice daily for 10 to 20 days. Therefore, the total dose recommended ranges between 5 grams and 20 grams of metronidazole. The pH of this formulation was tested by an independent laboratory to be pH 6.1.

So far as known, no one has heretofore formulated or used metronidazole for intravaginal treatment at the physiological pH of the vagina (that is, a pH in the range of about 3 to about 4.25). In addition, no one has successfully treated BV or trichomonasis with less than multiple gram doses of metronidazole.

The need for a safe and effective treatment for vaginitis such as bacterial vaginosis and trichomoniasis which can eliminate the invading organisms at a low, safe dose and provide the necessary vaginal environment for growth and maintenance of lactate-producing organisms remains.

With a view to meeting this need, the present invention is directed to a gel composition as defined in claim 1, with preferred formulations being presented in the subsidiary claims. As such, the invention provides a safe and effective, relatively low-dose treatment of a human vagina which is afflicted with BV or trichomoniasis, hereinafter collectively referred to as vaginitis. The invention also obviates the need for oral or intravenous administration of metronidazole for BV or for trichomoniasis, which administration can lead to undesirable side effects, as indicated above.

The gel composition of this invention can be introduced into an afflicted vagina, not only to provide an effective relatively low-dose treatment of vaginitis, but also to promote the beneficial and effective re-establishment of normal vaginal microflora, such as Lactobacilli and aerobic Streptococci. Thus, the gel composition provides not only an effective vaginitis treatment, but also a safe treatment since it leaves the treated vagina in a normal condition able to cope with, and resist, future microorganism infections. So far as now known, no other existing vaginitis treatment offers such an advantage.

It is true that EP-A-0 024 023 discloses tablets which contain metronidazole for intravaginal application, while EP-A-0 257 007 discloses creams and gels for treating conditions of the vagina, but neither of these two prior Specifications contemplates such treatment with such low metronidazole-dose compositions as the present invention.

A prolonged, substantially uniform and controlled release rate of metronidazole from the treating composition in the vaginal canal is provided by the compositions of the present invention.

In a presently preferred mode of practising this invention, a composition containing metronidazole as the sole active ingredient together with a buffer system capable of providing a buffered pH value in the range of 3.75 to 4.25 is administered intravaginally to a patient afflicted with BV and/or trichonioniasis at a total dose rate of 375 milligrams of metronidazole, administered in unit doses of at least 20 milligrams each one to three times daily over a period of three to ten days. This dose is approximately ten-fold less than that previously employed for effective therapy with metronidazole. This reduced dose rate is believed to be related to the difference in pH adjustment and maintenance.

Numerous other advantages and features of the present invention will become readily apparent from the following description of the preferred embodiments of the invention, the accompanying examples, the drawings, and the appended claims.

Brief Description of the Drawings

In the figures forming a part of the disclosure:
FIG. 1 is a graph illustrating the buffering capacity of a gel composition of the type used in the practice of this invention when titrated with a relatively dilute strong base; and
FIG. 2 is a graph illustrating the buffering capacity of the gel composition of FIG. 1 when titrated with a relatively concentrated strong base.

Description of Preferred Embodiments

While this invention is susceptible to embodiment in many different forms, preferred embodiments of the invention are described hereinbelow in detail. It should be understood, however, that the present disclosure and the embodiments described herein are to be considered as exemplifications of the principles of this invention and are not intended to limit the invention.

The present invention is practiced by introducing into such an afflicted vagina a therapeutically effective amount of a buffered formulation of metronidazole, such as hereinbelow described and exemplified. The term "vagina" as used herein is intended to be inclusive of the vaginal region generally, including also the vulva and the cervix. Also, the term "afflicted vagina" or "vaginitis" as used herein is intended to be inclusive of bacterial vaginosis (BV), trichomoniasis, and the causative microorganisms selected from protozoa, anaerobic bacteria, and mixtures thereof.

The quantity of metronidazole intravaginally introduced as a single or unit dose can vary widely, depending upon many variables, such as the age and physical condition of the patient, the extent of the patient's affliction, the frequency of administration, and the like.

The term "unit dose" or "unit dosage form" as used in the specification and claims refers to physically discrete units of such gel composition suitable for use as unitary dosages by human female subjects. Each unit contains a predetermined quantity of metronidazole calculated to produce the desired therapeutic effect in association with the required pharmaceutical vehicle. The exact novel unit dosage form(s) of the invention to be used for any given patient is/are dictated by, and directly dependent on (a) the unique characteristics of the metronidazole compositions and the particular therapeutic effects to be achieved, and (b) the characteristics, especially the release rate of metronidazole from the particular composition contemplated for the intended therapeutic use, as disclosed in detail in the present specification, these being features of the present invention.

In practising this invention, a presently preferred technique is to extrude a gel composition through a tubular applicator from a storage vessel, such as a syringe or squeezable tube into the afflicted vagina. The volume of gel composition so contained within a single such vessel is conveniently and preferably selected so as to constitute a single dose, or two doses, or the like, so as to facilitate administration of a desired controlled dose to a patient. The storage vessel is initially sealed, but is opened at the time of use. If more than a single dose is present, the vessel is preferably resealable by a suitable closure means.

Another presently preferred technique is to employ a single use packet (such as a small envelope-like structure, or the like) containing an intended single unit dose. The packet is initially sealed, but is opened at the time of use by tearing, cutting, or the like at a desired or planned location in the packet after which the packet is manually squeezed so that the contents are directly administrable as desired.

The dose or total quantity of metronidazole contained in a unit dose in a gel vehicle is in the range of 20 to 40 mg. Such a dose can be administered one to three times daily (that is, at spaced intervals in a 24 hour period) over a period of three to ten days. In its gel form, a daily dose of 80 mg is sufficient. The usual total dose for compositions of the present invention is in the range of 300 mg to 5,000 mg. A presently preferred administration procedure is to employ a unit dose of 5 grams of gel (delivering a dose of 37.5 mg of metronidazole) administered twice daily for a period of five days, thereby to deliver a total dose of 375 mg. Those skilled in the art will appreciate that the foregoing dose levels are provided illustratively, and that higher and lower dose levels can be employed without departing from the present invention.

Such doses are significantly lower than the comparable 7 gram dose (500 mg b.i.d. employed for 7 days, the standard BV dosage) as currently utilized and recommended by CDC. The low daily dose of the particularly preferred gel composition directly applied to the site of activity decreases the risks of dose related side effects and potential systemic activity. The effectiveness of this novel, low dose therapy is believed to be related to the combination of site specificity, controlled release, pH adjustment, control of vaginal environment, and provision for reestablishment of necessary normal vaginal flora, i.e., lactate producing organisms.

The active ingredient in the present composition is 1-(2-hydroxyethyl)-2-methyl-5-nitroimidazole (metronidazole). This drug is described in US-A-2,944,061 (Jacob et al.), and is commercially available.

The term "metronidazole" as used in this specification and claims includes not only 1-(2-hydroxyethyl)-2-methyl-5-nitroimidazole, but also those analogs and derivatives of metronidazole (salts, esters, etc.) which are soluble in the aqueous or oil phases of the compositions described herein and which exhibit therapeutic activity when applied as taught by the present invention. A physiologically tolerable medium in gel form is utilized as the delivery vehicle for metronidazole.

The term physiologically tolerable medium" as used herein refers to one or more viscous-to-solid gel materials which are non-irritating to the vaginal region. While a given such medium in a presently contemplated composition can be comprised of a single material, a plurality of components can comprise such a medium as well. Examples of components include water, oil, surfactants, preservatives, penetration enhancers, preservatives, and the like, such as hereinbelow described and illustrated. For purposes of avoiding problems of pooling and running the physiologically tolerable medium is preferably characterized by a viscosity at ambient conditions (e.g., 25°C, 760 mm Hg) with said metronidazole and also said buffer system dissolved and/or dispersed therein which is at least sufficient to maintain a product composition of this invention in a non-flowable state.

The term "buffer system" or "buffer" as used herein has reference to a solute agent or agents which, when in water solution, stabilize such solution against a major change in pH (or hydrogen ion concentration) when acids or bases are added thereto. Solute agent or agents which are thus responsible for a resistance to change in pH from a starting buffered pH value in the range above indicated are well known.

For example, a pH of 4.024 can be obtained with a solution of 0.05 M acid potassium phthalate. Similarly, a pH value of 4.0 can be achieved with an acetic acid-sodium acetate buffer. Also, a pH value of 4.0 can be achieved with, for example, 50 ml of 0.1 molar potassium hydrogen phthalate plus 0.1 ml of 0.1 M HCl, and a pH value of 4.1 can be achieved with, for example, 50 ml of 0.1 M potassium hydrogen phthalate plus 1.3 ml of 0.1 M NaOH. Various other buffers for achieving the desired pH values are also available, for example, DL-valine (pH 4.0). Virtually any pharmaceutically acceptable buffer system can be used which will achieve a pH in the range indicated for topical applications.

Metronidazole can be formulated into buffered gels made with gelling agents. Some examples of these gelling agents are:

Carboxyvinyl polymers — cross-linked acrylic acid polymers, e.g., those commercially available from B.F. Goodrich Co., Akron, Ohio, under the designation CARBOPOL™.

The examples below exemplify these systems, but those skilled in the art will appreciate that substitutions, additions and/or omissions of the specified components can be made. A listing below exemplifies alternate components that could be incorporated in these examples:

Surfactants

As above indicated, the buffered formulations of this invention can contain one or more surfactants. Suitable surfactants include anionic, cationic, amphoteric and nonionic surfactants which are pharmaceutically acceptable in topical applications. Any one or more surfactants having the above characteristics can be used. Representative examples of suitable surfactants which can be used in the formulations of this invention are described in Martin and Cook, Remington's Practice of Pharmacy, 12th edition, 1961, pp. 219-226, R.G. Harry, Cosmetics: Their Principles and Practices, (1965), pp. 396-398 and 413-417, and E. Sagarin, Cosmetics Science and Technology, (1957), pp. 328-333, 1060-1063 and 1254. Representative surfactants which are suitable include:

A. Anionic agents

1. Sodium, potassium and ammonium soaps derived from fatty acids having from 10 to 22 carbon atoms; and polyvalent metal (magnesium, calcium, zinc, aluminum and lead) soaps derived from fatty acids having from 10 to 22 carbons.

2. Amine soaps derived from fatty acids having from 10 to 22 carbons and primary, secondary and tertiary amines, such as monoethanolamine, diethanolamine and triethanolamine, and cyclic amines, such as morpholine. An example is triethanolamine stearate.

3. Rosin soaps, such as sodium salts of rosin acids, e.g., abietic acid.

4. Alkali metal salts of sulfate compounds which can be represented by the formula $ROSO_3H$ wherein the R group represents an organic moiety, such as, for example, a fatty alcohol residue having up to 22 carbons. Examples include sodium lauryl sulfate, sodium cetyl sulfate, sodium monolauryl glyceryl sulfate, an oil such as sulfated castor, olive, teaseed, neat's foot cottonseed, rape seed, corn and rice oil.

5. Alkali metal salts of sulfonated compounds which can be represented by the formula $RSO_3H$ wherein the R group can have from 8 to 22 carbons. These include alkane sulfonates, such as dioctyl sodium sulfosuccinate, oxyethylated alkylaryl sulfate, alkyl aromatic sulfonates such as sodium isopropylnaphthalenesulfonate, sodium dodecylbenzenesulfonate and sodium sulfonaphthylstearate.

B. Cationic agents

1. Amine salts (e.g., hydrochlorides and acetates) derived from straight chain fatty amines having from 8 to 18 carbons. An example is octodecylamine hydrochloride.

2. Quaternary ammonium salts formed by alkylation of fatty amines with methyl chloride, dimethylsulfate and benzylchloride. These compounds can be represented by the formula [RR'R''R'''N]Y wherein each of R, R', R'', R''' is a long chain aliphatic group of from 8 to 22 carbons or a fatty acid amide residue; a short aliphatic group, such as methyl, ethyl, or propyl, an aromatic group, such as a phenyl or benzyl radical; or a heterocyclic group, such as pyridine or piperidine residue; and Y represents an inorganic or lower organic cation, such as chloride, bromide or acetate radical. Examples include triethanolamine stearate, cetyl trimethyl ammonium bromide, benzalkoniumchloride.

C. Nonionic agents

1. Ethers, such as condensation products of alkylphenols with from 6 to 20 moles of ethylene oxide, such phenols being monoalkylated, dialkylated or polyalkylated with alkyl side chains having from 5 to 18 carbons each, and the corresponding naphthalene or diphenyl compounds. Examples include polyoxyethylene, polyoxyethylene-polyoxypropylene copolymers.

2. Esters, such as compounds which can be represented by the formula RCOOR' wherein R is a long hydrocarbon chain derived from a fatty acid having from 12 to 22 carbons, and R' is derived from a polyhydric alcohol. Examples include glyceryl monostearate, diethylene glycol monolaurate, sorbitan fatty acid esters derived, for example, from lauric, palmitic, stearic and/or oleic acids.

3. Ether-esters wherein polyoxyethylene chains are found with an unreacted hydroxy group of esters of fatty acids and polyhydric alcohols.

4. Fatty acid amides, such as lauroyl diethanolamide.

D. Ampholytic agents

1. Surfactants, such as those having amino and carboxy groups. Examples include dodecyl B-alanine, imidazoline derivatives such as the so-called "Miranols".

2. Surfactants containing amino and sulfuric acid or sulfonic acid groups formed by condensing an alkanesulfonamide with formaldehyde and methyltaurine.

Suitable representative surfactants from the above indicated four general classes include sorbitan trioleate, sorbitan tristearate, sorbitan sesquioleate, glycerol monostearate, sorbitan monostearate, sorbitan monopalmitate, sorbitan monolaurate, polyoxyethylene lauryl ether, polyethylene glycol 400 monostearate, triethanolamine oleate, polyoxyethylene glycol 400 monolaurate, polyoxyethylene sorbitan monostearate, polyoxyethylenesorbitan monooleate, polyoxyethylene sorbitan monolaurate, sodium oleate, potassium oleate, sodium lauryl sulfate, lauroyl imidazoline, sodium dodecylbenzene sulfonate, sodium monoglyceride sulfate, sodium alkaralkyl polyglycol sulfate, sodium oleyl taurate, sodium dioctyl sulfosuccinate, lauryl polyglycol, ether, sodium dibutylnaphthalenesulfonate, alkyl phenol polyglycol ether, sorbitan monolaurate polyglycol ether, sulfonated castor oil, tall oil polyglycol ester, alkyl dimethyl benzylammonium chloride, alkyl naphthalene pyridinium chloride, cetyl dimethyl ethylammonium bromide, alkyl dimethyl chlorobenzylammonium chloride, dibutyl phenyl phenol sulfonate, ester of colaminoethylformyl methyl pyridinium chloride, sulfonated methyl oleylamide, sorbitan monolaurate polyglycol ether, polyglycol oleate, sodium lauryl sulfoacetate, sodium 2-ethylhexanol sulfate, sodium 7-ethyl-2-methylundecanol-4 sulfate, sodium 3,9-diethyltridecanol-6 sulfate, sodium lauryl and myristyl collamide sulfonate and N-(sodium sulfoethyl) oleamide.

Preservatives

As above indicated, the buffered compositions of this invention can contain suitable bacterostats, preservatives, inhibitors, such as methyl, ethyl, propyl, and butyl esters of parahydroxybenzoic acid, propyl gallate, sorbic acid and its sodium and potassium salts, propionic acid and its calcium and sodium salts, "Dioxin" (6-acetoxy-2,4-dimethyl-m-dioxane), "Bronopol" (2-bromo-2-nitropropane-1,3-diol) and salicylanilides such as disbromosalicylanilide, tribromosalicylamilides, "Cinaryl" 100 and 200 or "Dowicil" 100 and 200 (Cis isomer of 1-(3-chloroallyl-3,5,7-triaza-1-azanidadamantane chloride), hexachlorophene, sodium benzoate, citric acid, ethylene diaminetetraacetic acid and its alkali metal and alkaline earth metal salts, butyl hydroxyanisol, butyl hydroxytoluene, phenolic compounds such as chloro- and bromocresols and chloro- and bromo- oxylenols, quaternary ammonium compounds like benzalkonium chloride, aromatic alcohols such as phenylethyl alcohol, benzyl alcohol, etc., chlorobutanol, quinoline derivatives such as iodochlorhydroxyquinolin, and the like.

Other Adjuvants/Cosolvents

Other adjuvants which can be incorporated into a composition of this invention includes waxes, such as beeswax, spermaceti, paraffin waxes, and fatty acids, alcohols and amides having from 10 to 22 carbons.

Monohydric alcohols can be used, such as those having from 1 to 22 carbons per molecule, such as methanol, ethanol, propanol, isopropanol, butanol, hexanol, cetyl alcohol and stearyl alcohol.

Dihydric and polyhydric alcohols can be used, such as those having from 2 to 22 carbons per molecule, such as propylene glycol, glycerin, hexanetriols, such as 1,2,6-hexanetriol, sorbitol, 1,3-butanediol and 2,3-butanediol.

Polyethylene glycols and polypropylene glycols can be used, such as those having molecular weight in the range of about 100 to about 20,000.

Esters of aliphatic monobasic and dibasic acids can be used, such as those having from 2 to 22 carbons per molecule, with (a) monohydric alcohols having from 1 to 20 carbons per molecule, (b) di- and polyhydric alcohols having from 2 to 20 carbons per molecule, and (c) sugar alcohols. Examples include isopropyl myristate, myristyl myristate, cetyl stearate, methyl stearate, isopropyl sebacate, methyl sebacate, sucrose monolaurate, sucrose monostearate, and the like.

Sterols, such as cholesterol.

Buffers

In general, and as above indicated, buffers for the present compositions include any physiologically acceptable organic acid (and its corresponding salt), either liquid or solid (depending upon application), having a pKa around 3 to 5 including acetic, fumaric, lactic, citric, propionic, lactic, malic, succinic, and tartaric acids.

Gases

Compositions of this invention can contain air or some other medically/pharmaceutically/cosmetically acceptable gas which is emulsified in a liquid phase of such composition to provide a foam.

Illustrative Buffered Compositions of Metronidazole

A composition of the invention advantageously comprises, in general, at least 0.1 weight percent metronidazole, based on the total weight of the composition. Preferably metronidazole is present in an amount of 0.25% to 1.0%, and more preferably 0.75% by weight, based on the total weight of the composition. Typically, a composition contains not more than about 3 percent metronidazole. Larger and smaller contents of metronidazole can be used without departing from this invention.

Substantially oil-free, aqueous compositions containing metronidazole, in which this drug is solubilized in a single-phase aqueous gel, are a preferred class of embodiments used in the practice of this invention. The overall advantages of such aqueous gel compositions in treating BV have been discussed above, and are presented and illustrated in greater detail hereinbelow.

The actual concentration of metronidazole in any given such composition may vary, depending on variables such as the nature and degree of the BV being treated, the duration of the therapeutic treatment period contemplated, the size of the particular unit dose to be administered.

In the preferred compositions, metronidazole is in an aqueous solution of a high molecular weight polycarboxylated vinyl polymer. The polymer imparts a desirable viscous, gelled consistency to the composition when mixed with metronidazole and water. The preferred gel compositions contain at least about 95% by weight water, based on the total weight of the composition, and have the requisite degree of metronidazole concentration, and hence thermodynamic activity, for effective topical delivery and bioavailability of metronidazole in the vagina. The preferred gel compositions also have the requisite therapeutic activities as previously described.

The gel-forming polymer useful in compounding such compositions may be any suitable polyacrylic acid polymer which is hydrophilic and water-dispersible, has free carboxylic groups and relatively high base binding capacity, and forms a buffered gel of substantially uniform consistency when neutralized with a base. The molecular weight of the polymer is desirably in the range of about 1,250,000 and about 4,000,000 daltons. Suitable polyacrylic acid polymers include, but are not limited to, polyacrylic acid polymers slightly cross-linked with a polyalkenyl polyether, such as those commercially available from B.F. Goodrich, Cincinnati, Ohio, under the trademarks Carbopol 934, 940, 950 and 941. Carbopol 934P™ is a particularly preferred polymer for use in practicing this invention.

The polymer is present in an amount sufficient to cause gelling of a preferred composition, and to impart the desired viscous consistency to the resulting topical formulation. In addition and importantly, the polymer is used in concentrations that afford the buffering capacity and pH range that are necessary for this method. The metronidazole compositions advantageously comprise about 0.2% to about 7.0% by weight of the polymer, preferably about 0.5% to about 2.5%, and most preferably about 2.0% by weight of the polymer based on the total weight of the composition.

Aqueous solutions of these polymers form gels when neutralized with a base. Water-soluble bases which have been used to promote gelling of such polymers as the Carbopols™ include, for example, inorganic bases, such as an aqueous solution of ammonia, NaOH, and organic amine, e.g., alkylamines, such as methylamine and ethylamine, dialkylamines, trialkylamines, alkanolamines and dialkanolamines. Preferably a strong base is employed. The pharmaceutically effective component of the compositions of the present invention, metronidazole, is itself sufficiently basic to partially neutralize the acidic polymer in aqueous solution to the desired degree and to promote gelling.

Optionally, a preferred gel composition may further include a solubilizer, i.e., an agent that promotes penetration of the active drug into the microorganisms. Such solubilizers include, but are not limited to, dimethyl sulfoxide (DMSO) and propylene glycol, with the latter being preferred. The composition advantageously includes 1.0% to 50%, preferably 2% to 5%, and more preferably 3% by weight, of such solubilizer, based on the total weight of the composition.

Preservatives optionally may be incorporated into such gel compositions in an amount effective for inhibiting growth of microbes, such as yeast, molds, and bacteria during gel composition storage. Any conventional preservative may be used, with parabens being preferred. A mixture of methyl paraben and propyl paraben has been found to be particularly effective as a preservative. Most preferably, such a composition comprises 0.08% by weight of methyl paraben and 0.02% by weight of propyl paraben based on the total weight of the gel composition.

Ethylenediaminetetraacetic acid (EDTA) or one of its salts is commonly added to dermatological preparations, and may optionally be incorporated into the gel composition. EDTA chelates certain metals that may be present in the formulation, which is useful because some patients have adverse reactions to preparations containing metal impurities. The EDTA will also inhibit undesirable "browning" of the composition which may occur over time in compositions having a low pH value, e.g., a pH value of 3.0 to 4.5. Advantageously, a gel composition optionally further includes from 0.01% to 0.1%, preferably 0.05% by weight, of EDTA based on the total weight of the composition.

The final pH value of a gel composition may vary within a physiologically compatible range. Advantageously, the final pH value is a physiologically compatible, i.e., not harmful to biological tissue, adjusts and controls vaginal environment to normal, healthy range and is acidic. The pH value is 3 to 4.25, and preferably 3.75 to 4.25. Any suitable method of adjusting the pH value of aqueous solutions may be used. Advantageously, sodium hydroxide (NaOH) is added to the composition to bring the final pH value to the desired level. The gel compositions are more viscous at pH values that approach neutrality than at the more acidic pH values within the preferred range, i.e., viscosity increases as the polymer in the gel is neutralized to a greater degree, e.g.,

with NaOH.

The ingredients listed above may be combined in any order and manner that produces a composition comprising metronidazole dissolved in, and evenly dispersed throughout, a one-phase aqueous gel of the desired consistency and pH value. One suitable method of preparing such compositions involves preparation of an aqueous solution of the polymer, which will be called "Part A". Advantageously, this solution comprises the polymer in distilled water. A "Part B" is prepared comprising metronidazole. Mixing of Parts A and B results in gelling of the composition. The optional solubilizer and preservative(s) are preferably included in Part B. If EDTA is to be added to the formulation, it is preferably included in Part A. The pH value may then be adjusted to the desired level, e.g., by addition of NaOH.

The resulting homogeneous buffered gels having a pH in the range indicated possess the advantageous properties described above, including utilizing non-inflammatory and non-irritating ingredients. Higher specific activity of metronidazole results due to increased diffusion across membranes, release from the vehicle, and controlled pH. The result is greater therapeutic effectiveness using smaller amount of metronidazole. A formulation has a desirable consistency that prevents undesirable pooling and leaking of metronidazole. High concentrations of tissue-drying ingredients (e.g. alcohols and acetone), which are found, for example, in some preparations to promote drug solubility, are also avoided. Such ingredients at high concentration may excessively dry the patient's vaginal wall causing undesirable discomfort.

As indicated above, when such above described gel composition is introduced as described into an afflicted vagina, a prolonged and surprisingly uniform and regulated (controlled) release rate of metronidazole from the gel composition into the environment of the vagina is achieved. Pooling and running is minimized. The release rate or delivery is sustained for an extended period of time.

The release rate is such that the quantity of the drug which is delivered to vaginal tissues during the release period is at, or slightly above, a minimum therapeutically effective level.

The gel composition also has an unusual and very useful buffering capacity which, in addition to, and in coaction with, the desired bactericidal activity of the metronidazole, is desirable and important in achieving the therapeutic effectiveness that is associated with the practice of this invention. This combination allows for the therapeutic effectiveness of the novel low dose metronidazole formulation by adjusting and controlling the pH of the vaginal environment.

Thus, the gel compositions, as is characteristic of a buffered composition of the invention generally, resist changes in pH upon exposure in the use environment to an acid or a base. In the preparation of a gel composition as above explained herein, a strong base (e.g., sodium hydroxide) is preferably added to the Carbopol™ polymer (weak acid form). This neutralization thickens the formulation to produce the desired gel consistency. It also produces the mixture of components needed to produce a buffered system.

As the exemplary material hereinbelow presented indicates, when a portion of a gel formulation is titrated by a strong base (e.g., sodium hydroxide) successively using each of a concentrated solution of the base and a dilute solution of the base, such that the total volume of base is substantially increased (for example, doubled), it is found not only that there is a significant buffering effect inherent in the gel formulation, but also that there is very little effect on the gel formulation buffer strength as a result of dilution.

These results are significant for purposes of accomplishing topical treatment of, for example, BV by the practice of this invention. For one thing, these results show that the inherent dilution of a unit dose of gel composition which occurs in the vagina does not affect the ability of the gel composition to help prevent and to treat the undesirable alkalinization of the vaginal tissue caused by infections of the BV type. For another thing, these results show that vaginal tissue can be promoted to remain at a pH below about 4.5 which is desirable to inhibit BV organism activity, and to promote certain desirable and normal bacterial colonization and development, such as Lactobacilli, and the like. For still another thing, these results show that the prolonged release rate characteristics associated with the gel composition in the vagina are largely unaffected by unit dose dilution.

The practice of the present invention is demonstrated in the following examples. These examples are meant to illustrate the invention. Variations in the treating gel-compositions which do not adversely affect the effectiveness of metronidazole will be evident to one skilled in the art. For example, additional ingredients such as coloring agents may be included in the compositions as long as the resulting composition retains desirable properties, as described above. Unless otherwise indicated, each composition is prepared by conventionally admixing the respective indicated components together. Also, unless otherwise indicated, each composition is prepared using a buffer (buffer system) which in use provides a pH value in the range of 3 to 4.25.

EXAMPLE 1: Gel Preparation

A 30 kilogram batch of a composition of the present invention was prepared as follows. 600 grams of Carbopol 934P™ (2.0% by weight of the final weight of the composition) was dissolved in 16.5 liters of distilled water containing 15 grams of ethylenediamine-tetraacetic acid (EDTA) disodium dihydrate. Sufficient amount of 10 weight percent sodium hydroxide (NaOH) solution was added to bring the pH value to about 3.75 to 3.9. This aqueous polymer solution was called "Part A". "Part B" was prepared by mixing 900 grams of propylene glycol (3% by weight of the final weight of the composition), 24 grams of methyl paraben (0.08% by weight of the final weight of the composition) and 6.0 grams of propyl paraben (0.02% by weight of the final weight of the composition). The mixture was added to 225 grams of metronidazole dispersed in 11.4 liters of distilled water maintained at 50 C. Parts A and B were then mixed thoroughly and gelling of the composition resulted. A cold aqueous solution of NaOH was then used to adjust the final pH value to 4.0. Distilled water was then added to give the desired 30 kilogram final weight. The NaOH and water were thoroughly mixed into the viscous gel.

EXAMPLE 2: Aqueous Solutions or Suspensions

```
                        Composition I
                 (Buffered Metronidazole Gel
               Composition; Preferred Embodiment)
```

| Ingredient | Wt % |
|---|---|
| Metronidazole | 0.1 - 1 |
| "Carbopol 934P" | 1 - 2 |
| Edetate Disodium | 0.05 |
| Propylene Glycol | 0 - 15 |
| Methyl Paraben | 0.08 |
| Propyl Paraben | 0.02 |
| NaOH 10% solution (q.s.) | pH 3.75 - 4.25 |
| Water (q.s.) | 100 |

A composition constituted by the buffer system and the physiologically tolerable medium, but without metronidazole, is also useful as a vaginal acidifier. Such a composition is illustrated below.

EXAMPLE 3: The Buffering Effect of the Metronidazole Gel Formulation

To determine and demonstrate the effectiveness of the gel composition as a buffer, the following work was carried out:

Procedure:

The gel formulation delineated in Table I below was prepared by the procedure of Example 1 above except for sodium hydroxide addition as described herein, and such was then titrated by the addition of strong base. A titration was carried out on each of two separate batches of the formulation. In one case, the titrant was a concentrated aqueous solution of sodium hydroxide (2.5N). This solution increased the resulting total composition volume only about 8 cc. In the other case, a dilute solution of sodium hydroxide (0.1N) was used as the titrant, which resulted in a doubling of the resulting composition volume from about 100 cc to 200 cc. This procedure allowed an examination of the effects of dilution on the buffer strength of the product.

## TABLE I

### Metronidazole Gel Formulation

| Component | Percent W/W |
|---|---|
| Metronidazole | 0.75 |
| Propylene Glycol | 3.00 |
| Propyl Paraben | 0.02 |
| Methyl Paraben | 0.08 |
| Disodium EDTA | 0.05 |
| Carbopol 934-P | 1.60 |
| Sodium Hydroxide | a |
| Distilled Water (q.s.) | 100.00 |

[a]Sodium hydroxide was omitted from this formulation so that titration could be carried out.

Results:

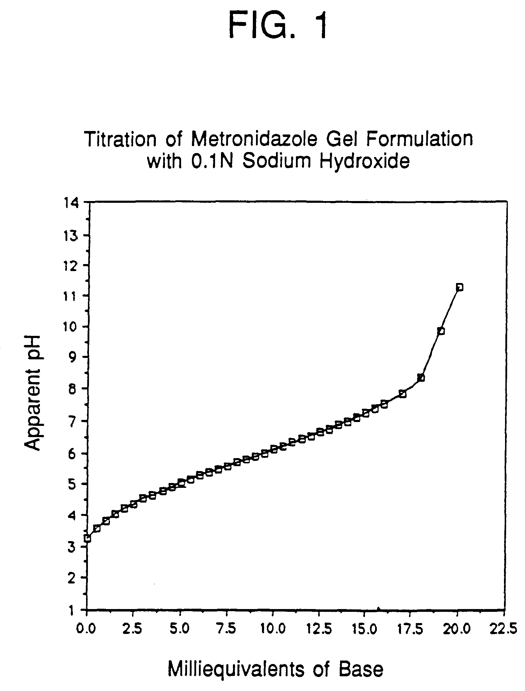

The titration data that resulted using the 0.1N sodium hydroxide is presented in Table II below and shown in accompanying FIGURE 1. The pH range over which there is significant buffering is from about pH 4 to 7.5. The slope in this region is 0.228. The reciprocal of the slope, 4.39, is the buffer capacity. This means that 4.39 mEq of base are needed to change the pH by one unit. The slope in the pH range from 4.05 to 4.92 is 0.285 and the buffer capacity in this region is slightly less at 3.51. The slope in the pH range from 4.92 to 6.89 is 0.213 and the buffer capacity is 4.69.

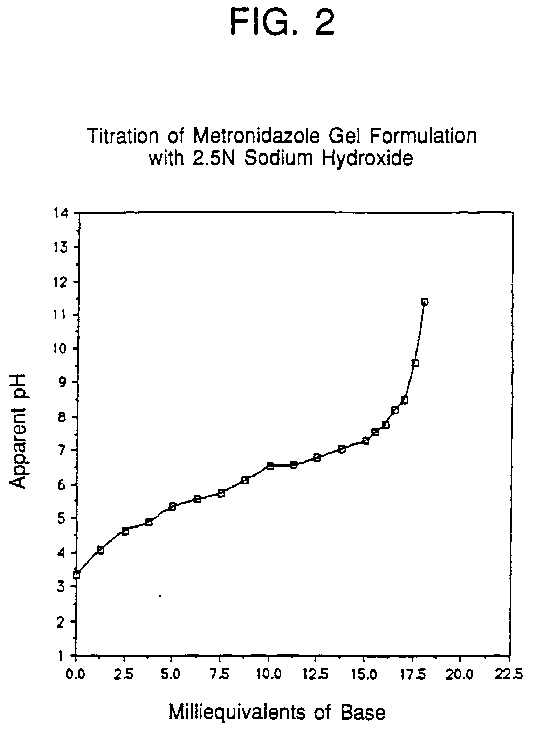

The titration data using the 2.5N sodium hydroxide is presented in Table III and shown in FIGURE 2. Again there is a significant buffering effect over a pH range of about 4 to 7.5. The slope of the titration curve in this region is 0.230 and the buffer capacity is 4.36. The slope from pH 4.08 to 4.89 is 0.324 and the buffer capacity is 3.09. The slope in the pH range from pH 4.89 to 6.79 is 0.220 and the buffer capacity is 4.55. This data is very similar to the titration data using the more dilute titrant.

Conclusions:

1. There is a significant buffering effect by the components of the metronidazole gel formulation over a pH range of 4 to 7.5.
2. There is very little effect on the buffer strength of the formulation upon dilution. This is significant since the formulation will become diluted when used, but will not lose its ability to help prevent and treat the alkalinization of the environment caused by infections of the type treated by metronidazole.

TABLE II

Titration Data Using 0.1N Sodium Hydroxide

| mEq of Base | pH | mEq of Base | pH |
|---|---|---|---|
| 0 | 3.27 | 10.5 | 6.20 |
| 0.5 | 3.57 | 11.0 | 6.33 |
| 1.0 | 3.83 | 11.5 | 6.43 |
| 1.5 | 4.05 | 12.0 | 6.55 |
| 2.0 | 4.22 | 12.5 | 6.67 |
| 2.5 | 4.37 | 13.0 | 6.77 |
| 3.0 | 4.56 | 13.5 | 6.89 |
| 3.5 | 4.65 | 14.0 | 7.01 |
| 4.0 | 4.77 | 14.5 | 7.14 |
| 4.5 | 4.92 | 15.0 | 7.28 |
| 5.0 | 5.07 | 15.5 | 7.43 |
| 5.5 | 5.17 | 16.0 | 7.55 |
| 6.0 | 5.29 | 17.0 | 7.89 |
| 6.5 | 5.39 | 18.0 | 8.36 |
| 7.0 | 5.48 | 19.0 | 9.85 |
| 7.5 | 5.58 | 20.0 | 11.26 |
| 8.0 | 5.68 | | |
| 8.5 | 5.79 | | |
| 9.0 | 5.89 | | |
| 9.5 | 6.00 | | |
| 10.0 | 6.11 | | |

## TABLE III

### Titration Data Using 2.5N Sodium Hydroxide

| mEq of Base | pH | mEq of Base | pH |
|---|---|---|---|
| 0 | 3.33 | 12.50 | 6.79 |
| 1.25 | 4.08 | 13.75 | 7.05 |
| 2.50 | 4.64 | 15.00 | 7.30 |
| 3.75 | 4.89 | 15.50 | 7.56 |
| 5.00 | 5.35 | 16.00 | 7.78 |
| 6.25 | 5.54 | 16.50 | 8.20 |
| 7.50 | 5.75 | 17.00 | 8.52 |
| 8.75 | 6.11 | 17.50 | 9.58 |
| 10.00 | 6.53 | 18.00 | 11.42 |
| 11.25 | 6.57 | | |

EXAMPLES 4 and 5: Clinical Trials: BV

To investigate the effectiveness of the method of this invention for the treatment of BV, the following clinical trials were conducted:

Two groups of human female patients were established. One group was treated for three days; the second group was treated for seven days.

All patients participating in these trials were preliminarily evaluated and were diagnosed to have BV based on positive tests in each patient of at least three of the four standard clinical test criteria employed for diagnosis of BV, as follows:

(1) clue cells comprise at least 20% of vaginal epithelial cells;
(2) homogeneous vaginal discharge;
(3) vaginal pH is greater than or equal to 4.7; and
(4) fishy amine odor appears upon addition of 10% KOH to vaginal discharge.

Each patient was otherwise found to be in good health based on a physical examination and stated medical history.

Only patients thus diagnosed to have solely BV were enrolled in these studies. Thus, patients who evidenced the presence of Candida or trichomoniasis vaginitis, whether concurrently with BV or not, were excluded, as were patients who were (a) involved in any concurrent antibiotic therapy for any condition within 14 days of the start of these studies, or (b) involved in the administration of any investigational drug within 30 days of the start of these studies. Also excluded were patients who had a history of hypersensitivity to metronidazole or to parabens, who were pregnant, who were nursing mothers, who were menstruating at the time of diagnosis, and/or who were unwilling to abstain from sexual intercourse during the treatment phase of the studies.

The vaginal gel used was prepared according to the procedure of Example 1 (above) and such contained 0.75 weight percent metronidazole. Five gram unit dose forms of the gel were administered on a twice daily basis in the morning and evening. Thus, each unit contained 37.5 mg of metronidazole.

Each patient was instructed to self-administer two unit doses daily, one in the morning, and one in the evening, for the assigned treatment period.

Each patient was examined at the end of her assigned treatment period. The presence of three of the above-indicated four standard clinical criteria for diagnosis of BV indicated a treatment failure. The lack of three of the above-indicated four standard clinical criteria for diagnosis of BV indicated a treatment success. Each patient was also examined for the presence of local or systemic adverse effects as a result of treatment.

In the three-day treatment, of the 10 patients treated, a 70% success rate was observed.

In the seven-day treatment, of the 11 patients treated, a 100% success rate was observed.

No local or systemic adverse effects were reported in any patients during these trials.

Data from the three-day treatment series is shown in Tables IV and V below (see Table Headings).

Data from the seven-day treatment series is shown in Tables VI and VII below (see Table Headings).

## TABLE IV

### Vaginal pH Values for Bacterial Vaginosis Patients Treated for 3 Days with 0.75% Metronidazole Gel

Vaginal pH

| Patient Number | (Baseline) Visit #1 | Visit #2 | Visit #3 | Visit #4 |
|---|---|---|---|---|
| 1 | 5.5 | 4.0 | 4.5 | 4.5 |
| 2 | 5.5 | 4.5 | 3.5 | 4.5 |
| 3 | 5.5 | 4.5 | 4.5 | Not taken |
| 4 | 5.5 | 4.5 | 4.0 | 4.0 |
| 5 | 4.5 | 4.0 | 4.0 | 4.0 |
| 6 | 4.5 | 4.5 | 4.5 | Terminated |
| 7 | 4.5 | 4.0 | 4.5 | Terminated |
| 8 | 5.5 | 4.0 | 4.0 | 4.0 |
| 9 | 5.0 | $3.75^{1}$ | $4.25^{2}$ | Not taken |
| 10 | 5.5 | 4.0 | 4.0 | 5.5 |

| n = 10 | n = 10 | n = 10 | n = 10 | n = 6 |
|---|---|---|---|---|
| | $\bar{x} = 5.15$ | $\bar{x} = 4.18$ | $\bar{x} = 4.18$ | $\bar{x} = 4.42$ |

[1]  Reported as range 3.5 to 4.0.

[2]  Reported as range 4.0 to 4.5.

Table V

Summary of Results on Bacterial Vaginosis
Patients Treated for 3 Days with 0.75% Metronidaozle Gel

| Patient Number | Visit #2 | | | Visit #3 | | | Visit #4 | | |
|---|---|---|---|---|---|---|---|---|---|
| | Days Since Baseline Visit | Treatment Success Or Failure | Days Since Last Visit | Days Since Baseline Visit | Treatment Success Or Failure | Days Since Last Visit | Days Since Baseline Visit | Treatment Success Or Failure | |
| 1 | 3 | Success | 17 | 20 | Success* | 14 | 34 | Success# | |
| 2 | 4 | Success | 23 | 27 | Success* | 8 | 35 | Success* | |
| 3 | 5 | Success | 11 | 16 | Success* | 18 | 34 | Success* | |
| 4 | 7 | Success | 10 | 17 | Success* | 12 | 29 | Success* | |
| 5 | 3 | Success | 14 | 17 | Success* | 18 | 35 | Success* | |
| 6 | 4 | Success | 14 | 18 | Failure* | -- | -- | -- | |
| 7 | 3 | Success | 14 | 17 | Failure* | -- | -- | -- | |
| 8 | 4 | Success | 14 | 18 | Success | 12 | 30 | Success* | |
| 9 | 4 | Success | 13 | 17 | Success* | 14 | 31 | Success* | |
| 10 | 3 | Success | 14 | 17 | Success* | 16 | 33 | Failure | |

| n = 10 | n = 10 | 10/10 Successes | n = 10 | n = 10 | 8/10 Successes | n = 8 | n = 8 | 7/10 Successes |
|---|---|---|---|---|---|---|---|---|
| | $\bar{x} = 4.0$ | | $\bar{x} = 14.4$ | $\bar{x} = 18.4$ | | $\bar{x} = 14.0$ | $\bar{x} = 32.6$ | |
| | (3-7 days) | | (10-23 days) | | | (8-18 days) | | |

\* Gram stain showed presence of Gram-positive rods indicative of Lactobacillus.

\# No Gram stain taken.

EP 0 404 376 B1

TABLE VI

Vaginal pH Values for Bacterial Vaginosis Patients
Treated for 7 Days with 0.75% Metronidazole Gel

Vaginal pH

| Patient Number | Visit #1 | (Baseline) Visit #2 | Visit #3 | Comments |
|---|---|---|---|---|
| 1 | 5.5 | --- | --- | Dropped |
| 2 | 5.0 | 4.5 | 4.5 | |
| 3 | 4.5 | 4.0 | 4.0 | |
| 4 | 5.5 | 4.0 | 4.0 | |
| 5 | 5.0 | 3.75[1] | 4.0 | |
| 6 | 5.5 | 3.75[1] | 3.5 | |
| 7 | 5.0 | 4.0 | --- | Dropped |
| 8 | 5.5 | 4.0 | 4.0 | |
| 9 | 5.0 | 3.5 | --- | |
| 10 | 5.5 | 4.0 | 4.5 | |
| 11 | 5.5 | 4.0 | 4.5 | |
| 12 | >5.5 | 4.5 | 5.0 | |
| 13 | 4.5 | 4.0 | 4.5 | |

n = 13    n = 13        n = 12            n = 10

$\overline{x} = 5.2$    $\overline{x} = 4.0$    $\overline{x} = 4.3$

[1] Reported as a range:  3.5 to 4.0.

17

TABLE VII

Summary of Results on Bacterial Vaginosis
Patients Treated for 7 Days with 0.75% Metronidazole Gel

| Patient Number | Age | Visit #2 Days Since Last Treatment Day | Treatment Success or Failure | Visit #3 Days Since Last Treatment Day | Treatment Success or Failure |
|---|---|---|---|---|---|
| 1 | 25 | 0 | Success | $--^{o}$ | --- |
| 2 | 20 | 0 | Success* | 8 | Success* |
| 3 | 22 | 0 | Success | 7 | Success* |
| 4 | 18 | 2 | Success* | 24 | Success[#] |
| 5 | 34 | 3 | Success* | 14 | Success* |
| 6 | 36 | 3 | Success | 17 | Success* |
| 7 | 20 | 10 | Success | $--^{+}$ | --- |
| 8 | 24 | 3 | Success* | 18 | Success* |
| 9 | 22 | 12 | Success* | 27 | Success* |
| 10 | 25 | 5 | Success* | 15 | Success* |
| 11 | 19 | 2 | Success* | 14 | Success |
| 12 | 21 | 1 | Success* | 13 | Success* |
| 13 | 23 | 1 | Success* | 15 | Success[#] |
| n = 13 | $\bar{x}$ = 23.8 years | $\bar{x}$ = 3.2 days | 13/13 = Success | $\bar{x}$ = 15.6 days | 11/11 = Success |
| | (18 to 36 years) | (0 to 12 days) | | (7 to 27 days) | |

[o] Dropped: Intrastudy treatment for chlamydia.
[+] Dropped: Intrastudy treatment for Candida.
* Gram stain showed presence of Gram-positive rods indicative of <u>Lactobacillus</u>.
[#] Gram stain not taken.

Example 6 - Clinical Trials:

Trichomonas Vaginalis Treatment

Using the gel composition of Example 1 above, two female patients who presented T. Vaginalis infections were each treated with a total dose of only 525 mg of metronidazole over a seven day period. Each patient administered a unit dose of 3.75 mg metronidazole two times daily.

One patient was considered a treatment success on the second follow-up examination at 11 days after the last treatment day.

The second patient was considered a treatment failure at the second follow-up examination at 18 days after the last treatment. Whether or not such failure was due to ineffective therapy and therefore a recurrence, or to reinfection from a sexual partner, was not determined.

Based on this encouraging limited data, it appears that the same combination of a low dose metronidazole delivered in a formulation that can adjust and maintain vaginal pH is useful in the treatment of T. vaginalis infections.

## Table VIII

## Summary of Results on Trichomoniasis
## Patients Treated for 7 Days with 0.75% Metronidazole Gel

| Patient Number (Age) | VISIT #2 Days Since Last Treatment Day | Treatment Success or Failure | VISIT #3 Days Since Last Treatment Day | Treatment Success or Failure |
|---|---|---|---|---|
| 14 (39) | 1 | Success | 18 | Failure |
| 15 (23) | 4 | Success | 11 | Success |

Bibliography:

Amsel R. Critchlow CW, Spiegel CA, Chen KCS, Eschenbach D, Smith K, Holmes KK. Edited by S.M. Finegold, W.L. Goerge and R.D. Rolfe. Comparison of metronidazole, ampicillin, and amoxicillin for the treatment of bacterial vaginosis (nonspecific vaginitis): possible explanation for the greater efficacy of metronidazole. Proceedings of the First U.S. Metronidazole Conference. Biomedical Information Corporation, 1982.

Amsel R, Critchlow CW, Spiegel CA, Chen KCS, Eschenbach D, Smith K, Holmes KK. "Comparison of metronidazole, ampicillin, and amoxicillin for treatment of bacterial vaginosis (nonspecific vaginitis): possible explanation for the greater efficacy of metronidazole." In proceedings of the First U.S. Metronidazole Conference: pp. 225-242. Edited by Finegold SM, George WL, and Rolfe RD. N.Y.: Biomedical Information Corporation, 1982.

Balsdon MJ, Pead L, Taylor GE, et al. "Corynebacterium vaginale and vaginitis: a controlled trial of treatment." Lancet 1, 501, 1980.

Bartlett JG, Polk BF. Bacterial flora of the vagina: quantitative study. Rev Infect Dis, (Suppl 1) 6, S67-S72, 1984.

Bistoletti P, Fredricsson B, Hagstrom B, Nord CE. "Comparison of oral and vaginal metronidazole therapy for nonspecific bacterial vaginosis." Gynecol Obstet Invest, 21, 144-149, 1986.

Centers for Disease Control. MMWR Supplement. "1985 STD treatment guidelines." Volume 34 (4S), October 18, 1985.

Charles D, Glover DD. Antimicrobial treatment of infectious vaginopathies. The Female Patient, 10, 25-42, 1985.

Davis B, Glover D, Larson B. Analysis of metronidazole penetration into vaginal fluid by reversed-phase high-performance liquid chromatography. Am J. Obstet Gynec, 149, 802-803, 1984.

Eschenbach DA, Critchlow CW, Watkins H, Smith K, Spiegel CA, Chen KCS, Holmes KK. "A dose-duration study of metronidazole for the treatment of nonspecific vaginosis." Scand J Infect Dis, (Suppl) 40, 73-80, 1983.

Eschenbach DA, Hillier S, Critchlow C, Stevens C, DeRoven T, Holmes KK. Diagnosis and clinical manifestations of bacterial vaginosis. Am J of Obstet & Gynecol, 158, 819-829, 1988.

Fouts AL, Kraus SJ. Trichomonas vaginalis: reevaluation of its clinical presentation and laboratory diagnosis. J Infect Dis, 141, 137-143, 1980.

Hager WD, Brown ST, Kraus SJ, et al. Metronidazole for vaginal trichomoniasis: seven-day vs. single-dose regimens. JAMA, 244, 1219-1220, 1980.

Hagstrom B, Lindstedt J. "Comparison of two different regimens of metronidazole in the treatment of nonspecific vaginitis." Scand J Infect Dis, (Suppl) 40, 95-96, 1983.

Hill, LVH, Embil JA. Vaginitis: current microbiological & clinical concepts. Can Med Assoc J, 134, 321-331, 1986.

Larsen B, Galask RP. Vaginal microbial flora: composition and influence of host physiology. Ann Intern Med, 96, 926-930, 1982.

Malouf M, Fortier M, Morin G, Dube J-L. "Treatment of Hemophilus vaginalis vaginitis." Obstet Gynecol, 57 (6), 711-714, 1981.

Mardh P, Soltesy LV. In vitro interactions between Lactobacilli and other microorganisms occurring in the vaginal flora. Scand J Infect Dis, (Suppl) 40, 47-51, 1983.

McCue JD. Evaluation and management of vaginitis. Arch Intern Med, 149, 565-568, 1989.

Mead PB, Thomson JL, Ledger WJ, Eschenbach DA. "Establishing bacterial vaginosis." Contemp OB/GYN, 27, 186-203, February 1986.

Peeters M, Piot P. Adhesion of Gardnerella vaginalis to vaginal epithelial cells: variables affecting adhesion and inhibition by metronidazole. Genitourin Med, 61, 391-395, 1985.

Pheifer TA, Forsyth PS, Durfee MA, Pollock HM, Holmes KK. "Nonspecific vaginitis: role of Haemophilus vaginalis and treatment with metronidazole." New Eng J Med, 298 (26), 1429-1434, 1978.

Purdon A, Hanna JH, Morse DL, et al. An evaluation of single dose metronidazole treatment for Gardnerella vaginalis vaginitis. Obste Gynecol, 64, 271, 1984.

Rein MF. Vulvovaginitis and cervicitis, in Mandell GL, Douglas RG, Bennett TE (eds). Principles and Practice of Infectious Diseases, edition 2, New York, John Wiley & Sons Inc., pp. 729-738.

Robbie MO, Sweet RL. "Metronidazole use in obstetrics and gynecology: A review. Am. J. Obstet. Gynecol. 145, 865-881, 1983.

Skavin A, Sylwan J. Vaginal Lactobacilli inhibiting growth of Gardnerella vaginalis, mobiluncus and other bacterial species cultured from vaginal content of women with bacterial vaginosis. Acta Path Microbiol Immunol Scand, Section B, 94, 399-403, 1986.

Staerfelt F, Gundersen TJ, Halsos AM, Barlinn C, Johansen AG, Norregaard KM, Eng, J. A survey of genital infections in patients attending a clinic for sexually transmitted diseases. Scand J Infect Dis, 40, 53-57, 1983.

Swedberg J, Steiner JF, Deiss F, Steiner S, Driggers DA. "Comparison of single dose versus one week course of metronidazole for symptomatic bacterial vaginosis." JAMA, 258 (8), 1046 1049, 1985.

Totten PA, Amsel R, Hales J, Piot P, Holmes KK. Selective differential human blood filagen media for isolation of Gardnerella vaginalis. J Clin Microbiol, 15, 141-147, 1982.

## Claims

1. A gel composition in unit dose form and suitable for intravaginal treatment of vaginitis, comprising :
   (a) metronidazole as the sole active ingredient dispersed in a gelled hydrophilic and water-dispersible polyacrylic acid polymer having free carboxylic acid groups and a molecular weight in the range of 1,250,000 to 4,000,000 daltons;
   (b) sufficient base to cause the composition to have a pH in the range of 3.75 to 4.25; and
   (c) an aqueous solvent for the metronidazole and the base;
   the unit dose containing 20 to 40 milligrams of metronidazole.

2. A gel composition according to claim 1, wherein the concentration of metronidazole present is at least 0.1% by weight, based on the total weight of the composition.

3. A gel composition according to claim 1 or claim 2, wherein the concentration of metronidazole is in the range of 0.25% to 1% by weight, based on the total weight of the composition.

4. A gel composition according to claim 1 or claim 2, wherein the concentration of metronidazole is 0.75% by weight, based on the total weight of the composition.

5. A gel composition according to any preceding claim, wherein the polymer is present in a range of 0.2% to 7% by weight, based on the total weight of the composition.

6. A gel composition according to any preceding claim, wherein the polymer is present in a range of 0.5% to 2.5% by weight, based on the total weight of the composition.

7. A gel composition according to any preceding claim, wherein the polymer is present in an amount of 2% by weight, based on the total weight of the composition.

8. A gel composition according to any preceding claim, wherein the gel composition includes a solubilizer.

9. A gel composition according to claim 8, wherein the solubilizer is propylene glycol and is present in an amount in the range of 2% to 5% by weight, based on the total weight of the composition.

10. A gel composition according to claim 9, wherein the propylene glycol is present in an amount of 3% by weight, based on the total weight of the composition.

11. A gel composition according to any preceding claim, wherein the gel composition includes a preservative.

12. A gel composition according to claim 11, wherein the preservative includes at least one paraben.

13. A gel composition according to claim 12, wherein the preservative consists essentially of methyl paraben present in an amount of 0.08% by weight and propyl paraben present in an amount of 0.02% by weight, based on the total weight of the composition.

14. A gel composition according to any preceding claim, wherein the gel composition further includes ethylenediamine-tetra acetic acid in an amount in the range of 0.01% to 0.1% by weight, based on the total weight of the composition.

15. A gel composition according to any preceding claim in the form of a unit dose which contains 37.5 milligrams of metronidazole.


**Patentansprüche**

1. Gelzusammensetzung in Form einer Einheitsdosis und zur intravaginalen Behandlung von Vaginitis geeignet, die umfaßt:
   (a) Metronidazol als einzigen Wirkstoff, dispergiert in einem gelierten, hydrophilen und in Wasser dispergierbaren Polyacrylsäurepolymer mit freien Carbonsäuregruppen und einem Molekulargewicht im Bereich von 1 250 000 bis 4 000 000 Dalton,
   (b) ausreichend Base, damit die Zusammensetzung einen pH-Wert im Bereich von 3,75 bis 4,25 hat, und
   (c) ein wäßriges Lösungsmittel für das Metronidazol und die Base, wobei die Einheitsdosis 20 bis 40 mg Metronidazol enthält.

2. Gelzusammensetzung nach Anspruch 1, in der die vorliegende Metronidazolkonzentration mindestens 0,1 Gew.-%, bezogen auf das Gesamtgewicht der Zusammensetzung, beträgt.

3. Gelzusammensetzung nach Anspruch 1 oder 2, in der die Metronidazolkonzentration im Bereich von 0,25 bis 1 Gew.-%, bezogen auf das Gesamtgewicht der Zusammensetzung, liegt.

4. Gelzusammensetzung nach Anspruch 1 oder 2, in der die Metronidazolkonzentration 0,75 Gew.-%, bezogen auf das Gesamtgewicht der Zusammensetzung, beträgt.

5. Gelzusammensetzung nach irgendeinem vorhergehenden Anspruch, in der das Polymer in einer Menge im Bereich von 0,2 bis 7 Gew.-%, bezogen auf das Gesamtgewicht der Zusammensetzung, vorliegt.

6. Gelzusammensetzung nach irgendeinem vorhergehenden Anspruch, in der das Polymer in einer Menge im Bereich von 0,5 bis 2,5 Gew.-%, bezogen auf das Gesamtgewicht der Zusammensetzung, vorliegt.

7. Gelzusammensetzung nach irgendeinem vorhergehenden Anspruch, in der das Polymer in einer Menge von 2 Gew.-%, bezogen auf das Gesamtgewicht der Zusammensetzung, vorliegt.

8. Gelzusammensetzung nach irgendeinem vorhergehenden Anspruch, die ein Mittel zum Löslichmachen einschließt.

9. Gelzusammensetzung nach Anspruch 8, in der das Mittel zum Löslichmachen Propylenglykol ist und in einer Menge im Bereich von 2 bis 5 Gew.-%, bezogen auf das Gesamtgewicht der Zusammensetzung, vorliegt.

10. Gelzusammensetzung nach Anspruch 9, in der das Propylenglykol in einer Menge von 3 Gew.-%, bezogen auf das Gesamtgewicht der Zusammensetzung, vorliegt.

11. Gelzusammensetzung nach irgendeinem vorhergehenden Anspruch, die ein Konservierungsmittel einschließt.

12. Gelzusammensetzung nach Anspruch 11, in der das Konservierungsmittel mindestens ein Paraben umfaßt.

13. Gelzusammensetzung nach Anspruch 12, in der das Konservierungsmittel im wesentlichen besteht aus Methylparaben, das in einer Menge von 0,08 Gew.-%, und Propylparaben, das in einer Menge von 0,02 Gew.-%, bezogen auf das Gesamtgewicht der Zusammensetzung, vorliegt.

14. Gelzusammensetzung nach irgendeinem vorhergehenden Anspruch, die ferner Ethylendiamintetraessigsäure in einer Menge im Bereich von 0,01 bis 0,1 Gew.-%, bezogen auf das Gesamtgewicht der Zusammensetzung, umfaßt.

15. Gelzusammensetzung nach irgendeinem vorhergehenden Anspruch, in Form einer Einheitsdosis, die 37,5 mg Metronidazol enthält.

## Revendications

1. Composition en gel se présentant sous la forme d'une dose unitaire et appropriée pour le traitement intravaginal de la vaginite, comprenant :
   (a) du métronidazole en tant qu'ingrédient actif unique, dispersé dans un acide polyacrylique polymère, gélifié, hydrophile et dispersable dans l'eau, ayant des groupes acide carboxylique libres et une masse moléculaire se situant dans la plage de 1 250 000 à 4 000 000 daltons ;
   (b) suffisamment de base pour amener la composition à présenter un pH se situant dans la plage de 3,75 à 4,25 ; et
   (c) un solvant aqueux pour le métronidazole et la base ;
   la dose unitaire contenant 20 à 40 milligrammes de métronidazole.

2. Composition en gel selon la revendication 1, dans laquelle la concentration de métronidazole présent est d'au moins 0,1% en poids, sur la base du poids total de la composition.

3. Composition en gel selon la revendication 1 ou la revendication 2, dans laquelle la concentration de métronidazole se situe dans la plage de 0,25% à 1% en poids, sur la base du poids total de la composition.

4. Composition en gel selon la revendication 1 ou la revendication 2, dans laquelle la concentration de métronidazole est de 0,75% en poids, sur la base du poids total de la composition.

5. Composition en gel selon l'une quelconque des revendications précédentes, dans laquelle le polymère est présent dans une plage de 0,2% à 7% en poids, sur la base du poids total de la composition.

6. Composition en gel selon l'une quelconque des revendications précédentes, dans laquelle le polymère est présent dans une plage de 0,5% à 2,5% en poids, sur la base du poids total de la composition.

7. Composition en gel selon l'une quelconque des revendications précédentes, dans laquelle le polymère est présent dans une quantité de 2% en poids, sur la base du poids total de la composition.

8. Composition en gel selon l'une quelconque des revendications précédentes, dans laquelle la composition en gel comprend un agent de solubilisation.

9. Composition en gel selon la revendication 8, dans laquelle l'agent de solubilisation est le propylène glycol et est présent dans une quantité se situant dans la plage de 2% à 5% en poids, sur la base du poids total de la composition.

10. Composition en gel selon la revendication 9, dans laquelle le propylène glycol est présent dans une quantité de 3% en poids, sur la base du poids total de la composition.

11. Composition en gel selon l'une quelconque des revendications précédentes, dans laquelle la composition en gel comprend un agent conservateur.

12. Composition en gel selon la revendication 1, dans laquelle l'agent conservateur comprend au moins un parahydroxybenzoate.

13. Composition en gel selon la revendication 12, dans laquelle l'agent conservateur consiste essentiellement en parahydroxybenzoate de méthyle présent dans une quantité de 0,08% en poids et en parahydroxybenzoate de propyle présent dans une quantité de 0,02% en poids, sur la base du poids total de la composition.

14. Composition en gel selon l'une quelconque des revendications précédentes, dans laquelle la composition en gel comprend en outre de l'acide éthylène diamine tétra acétique, dans une quantité se situant dans la plage de 0,01% à 0,1% en poids, sur la base du poids total de la composition.

15. Composition en gel selon l'une quelconque des revendications précédentes, se présentant sous la forme d'une dose unitaire qui contient 37,5 milligrammes de métronidazole.

# FIG. 1

Titration of Metronidazole Gel Formulation
with 0.1N Sodium Hydroxide

# FIG. 2

Titration of Metronidazole Gel Formulation
with 2.5N Sodium Hydroxide